# EUROPÄISCHE PATENTSCHRIFT

(11) **EP 3 835 759 B1**
(45) Veröffentlichungstag und Bekanntmachung des Hinweises auf die Patenterteilung: **30.08.2023**
(21) Anmeldenummer: 19214849.2
(22) Anmeldetag: 10.12.2019
(51) Int. Cl.: G01N 21/27, G01N 21/35, G01N 33/00, G01N 21/39

(54) **VERFAHREN UND GASANALYSATOR ZUR BESTIMMUNG DER KONZENTRATION EINES MESSGASES IN EINEM GASGEMISCH**
METHOD AND GAS ANALYSER FOR MEASURING THE CONCENTRATION OF A MEASUREMENT GAS IN A GAS MIXTURE
PROCÉDÉ ET ANALYSEUR DE GAZ PERMETTANT DE DÉTERMINER LA CONCENTRATION D'UN GAZ DE MESURE DANS UN MÉLANGE DE GAZ

(43) Veröffentlichungstag der Anmeldung: 16.06.2021
(73) Patentinhaber: Siemens Aktiengesellschaft, 80333 München (DE)
(72) Erfinder: Bitter, Ralf, 76356 Weingarten (DE); Depenheuer, Daniel, 67365 Schwegenheim (DE); Rupp, Simone Isabel, 76297 Stutensee (DE)
(74) Vertreter: Siemens Patent Attorneys

(56) Entgegenhaltungen:
- US-A1- 2007 081 162
- US-A1- 2012 113 426
- US-A1- 2017 003 218
- CHEN JIUYING ET AL: "Experimental study on mutual broadening coefficient between COand CO and its temperature dependence coefficient under high temperature", PROCEEDINGS OF SPIE; [PROCEEDINGS OF SPIE ISSN 0277-786X VOLUME 10524], SPIE, US, Bd. 9974, 19. September 2016 (2016-09-19), Seiten 99740V-99740V, XP060078681, DOI: 10.1117/12.2237238 ISBN: 978-1-5106-1533-5

## Beschreibung

Die Erfindung betrifft ein Verfahren zur Bestimmung der Konzentration eines Messgases in einem Gasgemisch.

Die Erfindung betrifft ferner einen Gasanalysator zur Bestimmung der Konzentration eines Messgases in einem Gasgemisch.

Eines der Hauptprobleme beim Einsatz absorptionsspektroskopischer Methoden zur Gasanalyse besteht im Vorhandensein von Quergasen, die Absorptionslinien des Messgases ganz oder teilweise überlagern und die Messung dadurch bei Nichtberücksichtigung verfälschen oder zumindest verschlechtern. Ein großer Quergaseinfluss kann die Nutzung von bestimmten Absorptionslinien auch komplett verhindern, was insbesondere dann problematisch wird, wenn bei bestimmten Gasen überhaupt nur wenige hinreichend starke Linien in einem technisch nutzbaren Wellenlängenbereich vorhanden sind. Um diese Linien trotzdem für Messungen nutzen zu können bzw. eine möglichst niedrige Nachweisgrenze erreichen zu können, ist es notwendig, den Einfluss der Quergaslinien auf die Messung möglichst stark zu verringern bzw. zu unterdrücken.

Ein besonderes Beispiel ist die Messung von Wasserstoff (H₂) in Gegenwart von Kohlendioxid (CO₂). So wird, wie z. B. in der WO 2019/117730 A1 gezeigt wird, eine der wenigen hinreichend starken Absorptionslinien von H₂, nämlich die Linie bei 2121,83 nm, von einer etwa fünfmal stärkeren CO₂-Linie in einer Entfernung von nur 0,06 nm stark überlagert. Das Problem verschärft sich, wenn, wie in industriellen Anwendungen häufig der Fall, mehr CO₂ als H₂ in dem zu analysierenden Gasgemisch vorhanden ist.

Es gibt eine Reihe von Ansätzen, um den Quergaseinfluss in spektroskopischen Messungen zu begrenzen. Dazu gehören:
- Messungen bei geringem Druck zur Verringerung der Druckverbreitung der Absorptionslinien, so dass die Mess- und Quergaslinie(n) schmaler und somit besser getrennt sind. Dabei wirkt sich jedoch nachteilig aus, dass mit dem geringeren Druck auch die absolute Anzahl an Molekülen in dem Messgas verringert wird und damit die Intensität der Absorptionslinie abgeschwächt wird.
- Optimierung der Modulationsamplitude in der Wellenlängenmodulationsspektroskopie (WMS) dahingehend, dass die Messlinie stark herausmoduliert wird, während die Quergaslinie(n) möglichst unterdrückt werden.
- Filterung der WMS-Daten nach Frequenzanteilen zur Diskriminierung und Unterscheidung unterschiedlich breiter Strukturen.

Der Einsatz dieser Methoden ist jedoch in vielen Fällen, wie z. B. auch bei der oben beschriebenen Überlagerung von H₂ durch CO₂, nicht ausreichend, um sehr kleine Messlinien vor einem großen Quergashintergrund genau vermessen zu können.

Aufgrund von Wechselwirkungen der Gasmoleküle mit Stoßpartnern kommt es mit zunehmender Dichte bzw. zunehmendem Druck des Gases zu einer Verbreiterung der Absorptionslinien (Druck- oder Stoßverbreiterung). Unter Ausnutzung der unterschiedlichen Druckverbreiterung von CO₂ und H₂ wird in der bereits erwähnten WO 2019/117730 A1 vorgeschlagen, die Messung von H₂ bei hohen statischen Drücken von 1 bar bis 5 bar vorzunehmen, wodurch die CO₂-Linie im Gegensatz zu der H₂-Linie stark druckverbreitert wird. Zum einen wird damit erreicht, dass die H₂-Messung durch den flacheren Untergrund weniger beeinflusst wird; zum anderen funktioniert die oben erwähnte Methode zur Verringerung des Quergaseinflusses durch Optimierung der Modulationsamplitude bei zunehmend unterschiedlichen Linienbreiten besser. Je nachdem, ob und welche weiteren Gase gemessen werden sollen, können intermittierend unterschiedliche Drücke eingestellt werden.

Aus der DE 196 49 343 A1 ist es bekannt, das Phänomen der Druckverbreiterung zur Erhöhung der differentiellen Messempfindlichkeit, d. h. der Änderung der Absorption in Abhängigkeit von der Konzentrationsänderung, zu nutzen. Insbesondere bei einer breitbandigen Messung über einen mehrere Absorptionslinien umfassenden Spektralbereich tragen selbst verhältnismäßig starke Konzentrationsänderungen nur wenig zu der im Wesentlichen durch die unterschiedlichen Absorptionen im Bereich der Spitzen der Absorptionslinien bestimmten Änderung der integralen Intensität bei, so dass sich eine nur geringe differentielle Empfindlichkeit ergibt. Während der niedrigere Druckwert dem Atmosphärendruck entsprechen kann, ist der höhere Druckwert um einen Faktor bis zu 5 so hoch, dass sich die Flanken benachbarter Absorptionslinien aufgrund der Druckverbreiterung überlappen. Das Ergebnis der bei dem niedrigen Druckwert vorgenommenen Einzelmessung mit geringer differentieller Empfindlichkeit kann als Referenz für die bei Druckverbreiterung erfolgende Einzelmessung dienen, die eine höhere differentielle Empfindlichkeit aufweist. Die Messung der Konzentration des Messgases in dem Gasgemisch erfolgt extraktiv in einer Messzelle mit einem steuerbaren Gaseinlassventil, einem steuerbaren Gasauslassventil und einem Kolbenverdichter zur Einstellung des Drucks in der Messzelle. Durch die Druckvariation wird die Konzentration bzw. Konzentrationsänderung des Messgases genauer bestimmt; das Problem von Quergaseinflüssen wird nicht angesprochen.

Aus der US 2017/003218 A1 ist es bekannt, die Messung der Konzentration eines Messgases, z. B. H₂S, in Gegenwart eines Quergases, z. B. CO₂, bei einem gegenüber dem Atmosphärendruck (760 Torr bzw. 1 atm) reduzierten Messdruck von z. B. 50 Torr vorzunehmen, so dass die Absorptionslinien des Messgases und des Quergases ausreichend voneinander getrennt sind. Die Zentralwellenlänge der Absorptionslinie des Messgases, die durch die Druckverringerung in ihrer Intensität stark geschwächt ist, wird durch Referenzierung auf die Wellenlängen von zwei wesentlich stärkeren Absorptionslinien des Quergases ermittelt. An der Stelle der so ermittelten Zentralwellenlänge wird die Amplitude der Absorptionslinie des Messgases gemessen und daraus dessen Konzentration bestimmt. Zur Kalibrierung der Amplitudenmessung kann bei unterschiedlichen vorgegebenen Messgas-Konzentrationen der Druck soweit abgesenkt werden, bis das Messgas nicht mehr detektierbar ist, wobei die Konzentrationswerte des Messgases und die Druckwerte mit entsprechenden Werten einer Nachschlagetabelle verglichen werden.

Der Erfindung liegt die Aufgabe zugrunde, die Bestimmung der Konzentration eines Messgases in Gegenwart eines spektral überlappenden Quergases zu verbessern, ohne dass dazu die Messung bei einem hohen statischen Druck erfolgen muss.

Gemäß der Erfindung wird die Aufgabe durch das in Anspruch 1 definierte Verfahren und durch den in Anspruch 7 angegebenen Gasanalysator gelöst.

Vorteilhafte Weiterbildungen der Erfindung sind in den Unteransprüchen angegeben.

Gegenstand der Erfindung ist somit ein Verfahren zur Bestimmung der Konzentration eines Messgases in einem Gasgemisch durch Messung der Absorption von Licht über den spektralen Bereich einer Absorptionslinie des Messgases, wobei das Gasgemisch ein Quergas mit mindestens einer weiteren Absorptionslinie enthält, die die Absorptionslinie des Messgases überlappt und ein im Vergleich zu dieser unterschiedliches Druckverbreiterungsverhalten aufweist, wobei
- der Druck des Gasgemischs variiert und die Messung der Absorption in Form von mindestens zwei Einzelmessungen bei unterschiedlichen Drücken des Gasgemischs durchgeführt wird,
- die Ergebnisse der Einzelmessungen mit von einem Kalibrierfeld bereitgestellten Referenzergebnissen verglichen werden, die zuvor durch Messung und/oder Simulation für unterschiedliche bekannte oder vorgegebene Drücke und Konzentrationen des Messgases und des Quergases ermittelt worden sind, und
- anhand des Vergleichs der mindestens zwei Einzelmessungen mit den Referenzergebnissen die Konzentration des Messgases in dem Gasgemisch bestimmt wird.

Gegenstand der Erfindung ist ferner ein Gasanalysator zur Bestimmung der Konzentration eines Messgases in einem Gasgemisch durch Messung der Absorption von Licht über den spektralen Bereich einer Absorptionslinie des Messgases, wobei das Gasgemisch ein Quergas mit mindestens einer weiteren Absorptionslinie enthält, die die Absorptionslinie des Messgases überlappt und ein im Vergleich zu dieser unterschiedliches Druckverbreiterungsverhalten aufweist,
- mit einer wellenlängenabstimmbaren Lichtquelle, deren Licht durch eine das Gasgemisch aufnehmende Messküvette auf einen Messdetektor mit nachgeschalteter Auswerteeinrichtung fällt, und
- mit einer Modulationseinrichtung, die zur wellenlängenabhängigen Abtastung der ersten Absorptionslinie die Wellenlänge des Lichts der Lichtquelle innerhalb periodisch aufeinanderfolgender Abtastintervalle variiert,
   wobei
- eine Druckeinstellanordnung vorhanden ist, die dazu ausgebildet ist, mindestens zwei unterschiedliche Drücke des Gasgemischs in der Messküvette einzustellen,
- die Auswerteeinrichtung ein Kalibrationsfeld mit durch Messung und/oder Simulation für unterschiedliche bekannte oder vorgegebene Drücke und Konzentrationen des Messgases und des Quergases ermittelten Referenzergebnissen für die Absorption enthält, und
- die Auswerteeinrichtung dazu ausgebildet ist, durch Vergleich der Ergebnisse von bei mindestens zwei unterschiedlichen Drücken des Gasgemischs durchgeführten Einzelmessungen der Absorption mit den von dem Kalibrierfeld bereitgestellten Referenzergebnissen die Konzentration des Messgases in dem Gasgemisch zu bestimmen.

Die Messung der Absorption von Licht über den spektralen Bereich der Absorptionslinie des Messgases kann je nachdem, welches Messverfahren verwendet wird, unterschiedlich erfolgen und dementsprechend zu unterschiedlichen Arten von Ergebnissen der Einzelmessungen und Referenzergebnissen führen. So kann bei der direkten Absorptionsspektroskopie (DAS) die Fläche unter der in dem Messignal enthaltenen Absorptionslinie bestimmt werden, die zur Gaskonzentration proportional ist. Die Bestimmung der Gaskonzentration lässt sich dadurch verbessern, dass eine physikalische Modellfunktion (Sollkurve) an die gemessenen Absorptionslinie (Istkurve) angefittet wird und die Fläche unter der Modellfunktion oder ein anderer Parameter der Modellfunktion bestimmt wird. Analog kann bei der Wellenlängenmodulationsspektroskopie (WMS) das demodulierte Messignal an eine im Idealfall zu erwartende und mittels eines Näherungsmodells analytisch beschriebene Modellfunktion des demodulierten Messsignals angefittet werden. Über einen zu der Konzentration der Gaskomponente proportionalen Parameter der Modellfunktion erhält man die Konzentration des zu messenden Gases. Bei den Ergebnissen der Einzelmessungen und den Referenzergebnissen kann es sich also um Werte, Parameter und Kurvenformen einer Absorptionslinie handeln.

Die vorliegenden Erfindung geht nun von dem Fall aus, dass die für die Messung verwendete Absorptionslinie des Messgases von mindestens einer Absorptionslinie des Quergases überlagert wird, und nutzt das unterschiedliche Druckverbreiterungsverhalten beider Absorptionslinien zur Diskriminierung der Beiträge des Messgases und des Quergases zu der gemessenen Absorption, um so die Störung der Konzentrationsmessung des Messgases durch das Quergas zu minimieren. Zu diesem Zweck erfolgt die Messung der Absorption in Form von mindestens zwei Einzelmessungen bei unterschiedlichen Drücken des zu analysierenden Gasgemischs. Die dabei erhaltenen Ergebnisse der Einzelmessungen werden mit Referenzergebnissen verglichen, die von einem Kalibrierfeld (Kalibriermatrix, o. dgl.) bereitgestellt werden. Bei den Referenzergebnissen kann es sich um Ergebnisse von Einzelmessungen handeln, die im Rahmen einer Kalibrierung bei unterschiedlichen bekannten Drücken und Konzentrationen des Messgases und des Quergases ermittelt worden sind und Stützstellen (Datensätze) bilden, zwischen denen für den Vergleich mit den mindestens zwei Einzelmessungen interpoliert werden kann. Die Referenzergebnisse können größtenteils auch über Simulationen erstellt werden. Diese können zur Sicherheit durch Messungen abgesichert werden, wobei jedoch vergleichsweise wenige Stützstellen ausreichen, die das gesamte Kalibrierfeld aufspannen. Aus dem Vergleich der bei den unterschiedlichen Drücken vorgenommenen Einzelmessungen mit den Referenzergebnissen wird die Konzentration des Messgases in dem Gasgemisch bestimmt.

Die Referenzergebnisse können unmittelbar in dem Kalibrationsfeld abgespeichert sein. Es können aber auch durch Messung und/oder Simulation ermittelte Basisdaten abgespeichert werden, aus denen mittels einer ebenfalls abgespeicherten Rechenvorschrift die Referenzergebnisse berechnet werden.

So können erste Referenz-Zwischenergebnisse für unterschiedliche Drücke und unterschiedliche Konzentrationen des Messgases ohne Anwesenheit des Quergases und zweite Referenz-Zwischenergebnisse für die unterschiedlichen Drücke und unterschiedlichen Konzentrationen des Quergases ohne Gegenwart des Messgases ermittelt werden und die Referenzergebnisse durch Addition der ersten und zweiten Referenz-Zwischenergebnisse berechnet werden. Dies erlaubt es die Anzahl der Stützwerte in dem Kalibrationsfeld angesichts der Vielzahl möglicher Kombinationen der Konzentrationen des Messgases und des Quergases gering zu halten. Die ersten und zweiten Referenz-Zwischenergebnisse können zusammen von dem Kalibrierfeld oder getrennt von Sub-Kalibrierfeldern bereitgestellt werden.

Schließlich können die Referenzergebnisse bzw. Referenz-Zwischenergebnisse jeweils als Absorptionen bei den unterschiedlichen Drücken oder alternativ als Absorptionen bei einem Druck und Änderung der Absorption bei Änderung des Druckes bereitgestellt werden. Dies ist dann von Vorteil, wenn die Änderung der Absorption proportional zur Änderung des Druckes ist, so dass das Kalibrierfeld mit weniger Stützstellen bzw. Datensätzen auskommt und im Messbetrieb mit anderen Drücken gearbeitet werden kann als die zur Erstellung des Kalibrierfeldes verwendeten Drücke.

Zur weiteren Erläuterung der Erfindung wird im Folgenden auf die Figuren der Zeichnung Bezug genommen; im Einzelnen zeigen:
- Fig. 1: ein Ausführungsbeispiel eines extraktiven Gasanalysators zur Durchführung des erfindungsgemäßen Verfahrens,
- Fig. 2: ein Beispiel für das unterschiedliche Druckverbreiterungsverhalten von H₂-Linien und CO₂-Linien,
- Fig. 3: ein Beispiel für das Kalibrierfeld, und
- Fig. 4: ein weiteres Beispiel für das Kalibrierfeld.

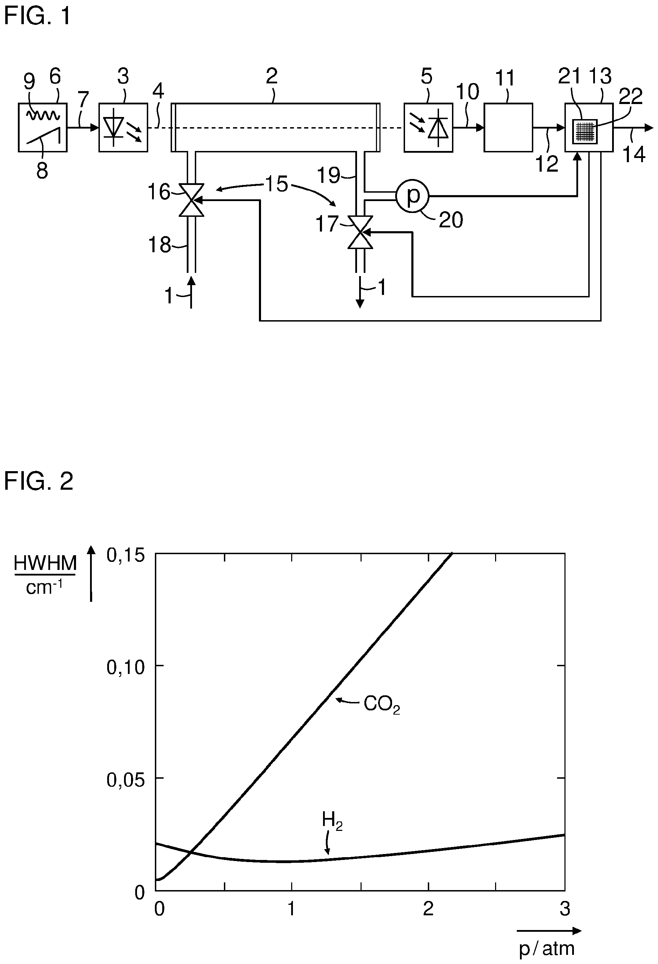

Bei dem in Fig. 1 in Form eines vereinfachten Blockschaltbildes gezeigten Gasanalysator handelt es sich um ein Laserspektrometer zur Messung der Konzentration mindestens einer interessierenden Gaskomponente (Messgas) eines Gasgemischs 1, das aus einem Prozess entnommen und durch eine Messküvette (Gaszelle) 2 geleitet wird. Das Spektrometer enthält eine durchstimmbare Lichtquelle 3 in Form einer Laserdiode, deren Licht 4 nach Durchstrahlen des Gasgemischs 1, auf einen Messdetektor 5 fällt. Die Laserdiode 3 wird von einer Modulationseinrichtung 6 mit einem Strom 7 angesteuert, wobei die Intensität und Wellenlänge des erzeugten Lichts 4 von dem Strom 7 und der Betriebstemperatur der Laserdiode 3 abhängen. Der Strom 7 wird periodisch entsprechend einer vorgegebenen, vorzugsweise rampen- oder dreieckförmigen Funktion 8 variiert, um mit der mehr oder weniger linear folgenden Wellenlänge des erzeugten Lichts 4 eine ausgewählte Absorptionslinie des Messgases wellenlängenabhängig abzutasten. Soll die Konzentration des Messgases auf Basis der Wellenlängenmodulationsspektroskopie (WMS) bestimmt werden, so werden der Strom 7 und damit die Wellenlänge des erzeugten Lichts 4 zusätzlich mit einer Frequenz im kHz-Bereich und kleiner Amplitude sinusförmig 9 moduliert.

Beim Durchstrahlen der Messzelle 2 wird das Licht 4 von den Gaskomponenten des Gasgemischs 1 wellenlängenabhängig absorbiert. Der Messdetektor 5 erzeugt in Abhängigkeit von der detektierten Lichtintensität ein Messsignal 10, das in einer Detektionseinrichtung 11 auf Basis der direkten Absorptionsspektroskopie (DAS) und/oder der Wellenlängenmodulationsspektroskopie (WMS) ausgewertet und digitalisiert wird. Dabei wird das Messsignal 10 für die DAS-Auswertung tiefpassgefiltert und normiert. Für die WMS-Auswertung wird das Messsignal 10 mittels Lock-in-Detektion bei der Frequenz der Sinusmodulation des Stromes 7 und /oder einer höheren Harmonischen dieser Frequenz phasensensitiv detektiert. Die Ergebnisse 12 der DAS- und/oder WMS-Auswertungen werden in einer Auswerteeinrichtung 13 zu einem Messergebnis 14 in Form der Konzentration des Messgases in dem Gasgemisch 1 ausgewertet.

Das zu analysierende Gasgemisch 1 enthält neben dem Messgas ein Quergas mit einer Absorptionslinie, welche die zu messende Absorptionslinie des Messgases überlappt. So kann es sich bei dem Messgas beispielsweise um H₂ handeln, dessen Konzentration in dem Gasgemisch 1 in Gegenwart von CO₂ durch Messen der H₂-Linie bei 2121,83 nm bestimmt werden soll. Wie eingangs bereits erwähnt, wird diese Linie von einer etwa fünfmal stärkeren CO₂-Linie stark überlagert.

Fig. 2 zeigt das unterschiedliche Druckverbreiterungsverhalten der H₂-Linie und der CO₂-Linie anhand der hier als halbe Breite bei einem halben Maximum (half width at half maximum) HWHM definierten Linienbreite der beiden Gase in Abhängigkeit von dem Druck p. Wie man sieht, ist die Druckabhängigkeit der Linienbreite von H₂ relativ gering und weist etwa bei Atmosphärendruck (1 atm) ein Minimum auf. Demgegenüber ist die Linienbreite von CO₂ vergleichsweise stark von dem Druck p abhängig und nimmt mit diesem praktisch proportional zu.

Zurück zu Fig. 1 weist der Gasanalysator eine Druckeinstellanordnung 15 mit zwei steuerbaren Ventilen 16, 17 auf, die in einer Gaszuleitung 18 zu und einer Gasableitung 19 von der Messküvette 2 angeordnet sind und von der Auswerteeinrichtung 13 gesteuert werden. Ferner ist die Auswerteeinrichtung 13 mit einem Drucksensor 20 verbunden, der den Druck p in der Messküvette 2 misst. Bei dem gezeigten Beispiel liegt das zu analysierende Gasgemisch 1 in dem Prozess, aus dem es entnommen wird, mit mehreren bar Überdruck vor, so wie z. B. bei der Messung von H₂ in Erdgas. In einem ersten Schritt wird das Gasgemisch 1 bei geöffneten Ventilen 16, 17 aus dem Prozess durch die Messküvette 2 geleitet. Danach werden zunächst das auslassseitige Ventil 17 und dann das einlassseitige Ventil 16 geschlossen, so dass das Gasgemisch 1 unter Überdruck in der Messküvette 2 vorliegt. Durch Öffnen oder Drosseln des Ventis 17 kann das Gasgemisch 1 gegen Atmosphäre entweichen, so dass sich auf diese Weise in der Messküvette 2 ein rampen- oder stufenförmiger Druckverlauf einstellen lässt, ohne dass eine Pumpe und eine aufwändige Regelung erforderlich sind. Wenn das Ventil 17 nur schalten aber nicht drosseln kann, kann eine hier nicht gezeigte Strömungsdrossel in Reihe mit dem Ventil 17 geschaltet werden. Es ist natürlich auch möglich, den Druck in der Messküvette 2 mittels einer Pumpe zu modulieren oder einzustellen, so wie dies z. B. aus der oben erwähnten DE 196 49 343 A1 bekannt ist.

Während der Messperiode steuert die Auswerteeinrichtung 13 die Druckeinstellanordnung 15 zur Veränderung des Druck des Gasgemischs 1 an und führt die Messung der Absorption in Form von mindestens zwei Einzelmessungen bei unterschiedlichen Drücken des Gasgemischs 1 durch. Die Ergebnisse 12 der Einzelmessungen werden mit Referenzergebnissen 22 verglichen, die zuvor durch Messung und/oder Simulation für unterschiedliche bekannte oder vorgegebene Drücke und Konzentrationen des Messgases und des Quergases ermittelt worden sind und mittels eines Kalibrierfelds 21 bereitgestellt werden. Anhand des Vergleichs der mindestens zwei Einzelmessungen 12 mit den Referenzergebnissen 22 wird die Konzentration des Messgases in dem Gasgemisch 1 bestimmt.

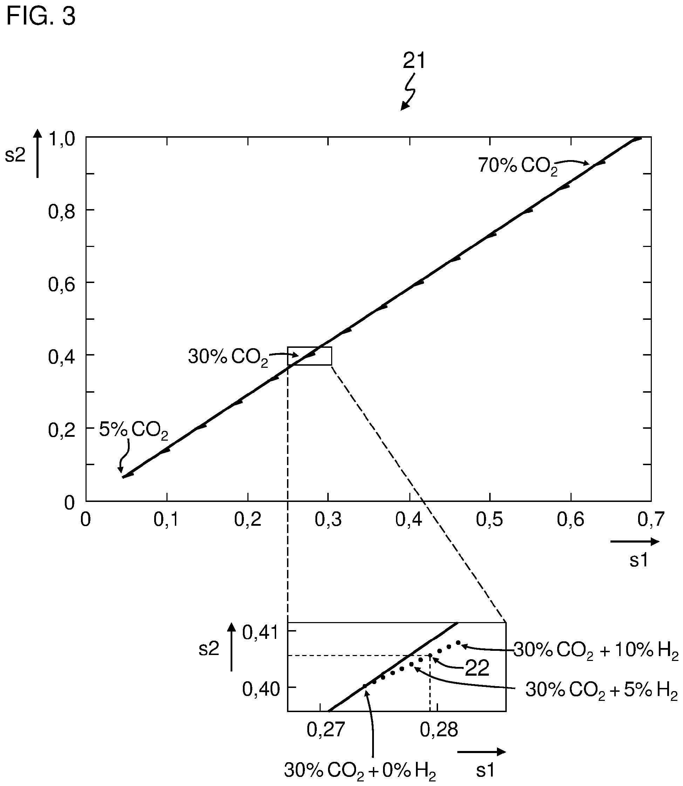

Fig. 3 zeigt ein Beispiel für das Kalibrierfeld 21, dessen Dimensionalität durch die Anzahl der unterschiedlichen Drücke bestimmt ist, bei denen die Einzelmessungen erfolgen und für die dementsprechend die Referenzergebnisse ermittelt werden. Im gezeigten Fall gilt das Kalibrierfeld 21 für die Messung von H₂ in Gegenwart von CO₂ bei zwei unterschiedlichen Drücken. Das Kalibrierfeld 21 ist daher zweidimensional, wobei auf der x-Achse s1 die über den spektralen Bereich der (durch die Anwesenheit von CO₂ gestörten) H₂-Linie messbare Absorption bei einem Druck von 1000 mbar und auf der y-Achse s2 die Absorption bei einem Druck von 1500 mbar bezeichnen. Die Referenzergebnisse 22 sind in Form von Tupeln (s1; s2) zusammengefasst, wobei jedes Tupel für eine vorgegebene oder bekannte Konzentration von H₂ in Gegenwart von einer vorgegebenen oder bekannten Konzentrationen von CO₂ im Rahmen einer Kalibration gemessen oder rechnerisch durch Simulation ermittelt wurde. Es reicht aus, einzelne Tupel (s1; s2) zu messen und die übrigen Tupel durch Interpolation zwischen den gemessenen Tupeln (Stützstellen) zu ermitteln. Bei dem gezeigten Beispiel sind die Werte von s1 und s2 auf den Messbereich normiert und daher dimensionslos. Wie oben bereits erwähnt, kann es sich bei Referenzergebnissen um Werte, Parameter oder Kurvenformen einer Absorptionslinie handeln. Im Falle der Kurvenformen bestehen die Komponenten s1, s2 der Tupel jeweils aus Datensätzen.

Bei dem gezeigten Beispiel beträgt das Referenzergebnis (Tupel) 22 für ein Gasgemisch mit einem 7-prozentigen H₂-Anteil und einem 30-prozentigen CO₂-Anteil:h
(s1; s2) = (0,2794; 0,4055).

Die Referenzergebnisse können auch durch (vektorielle) Addition von Referenz-Zwischenergebnissen gebildet werden, die einmal für H₂ ohne Beisein von CO₂ und das andere Mal für CO₂ ohne Beisein von H₂ ermittelt und in dem Kalibrierfeld 21 oder getrennten Sub-Kalibrierfeldern abgespeichert werden.

Im Falle des oben angegebenen Beispiels für ein Gasgemisch mit einem 7-prozentigen H₂-Anteil und einem 30-prozentigen CO₂-Anteil werden dann ein erstes Referenz-Zwischenergebnis (s11; s21) für ein CO₂-freies Gasgemisch mit einem 7-prozentigen H₂-Anteil (0 % CO₂ + 7 % H₂) und ein zweites Referenz-Zwischenergebnis (s12; s22) für ein H₂-freies Gasgemisch mit einem 30-prozentigen CO₂-Anteil (30 % CO₂ + 0 % H₂) ermittelt und beide Referenz-Zwischenergebnisse addiert:
(s1; s2) = (s11; s21) + (s12; s22) = (s11 + s12; s21 + s22).

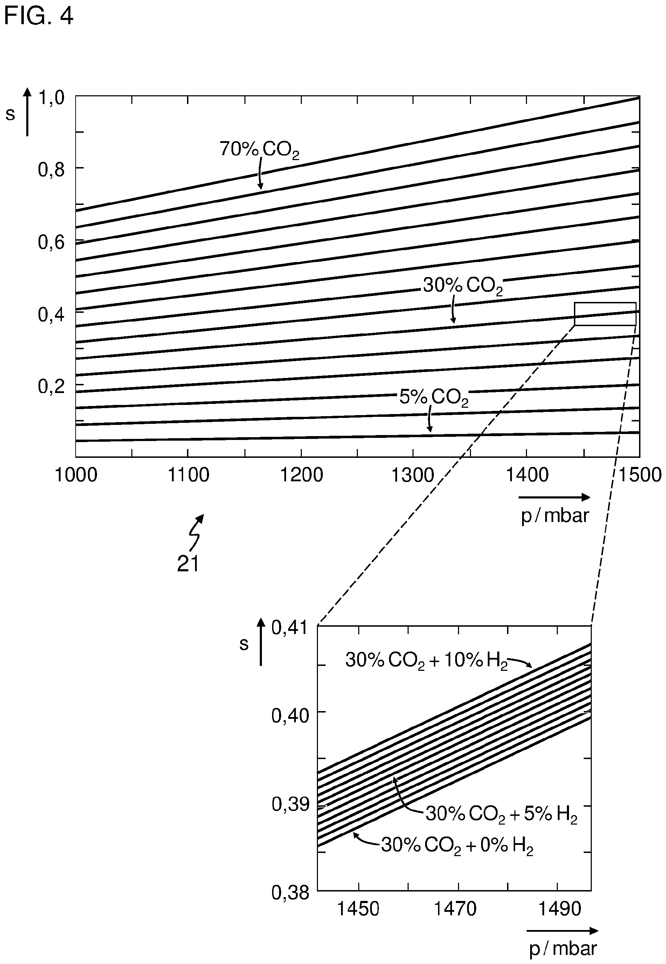

Fig. 4 zeigt ein weiteres Beispiel für das Kalibrierfeld 21, bei dem entlang der x-Achse der Druck p und auf der y-Achse die über den spektralen Bereich der (durch die Anwesenheit von CO₂ gestörten) H₂-Linie messbare Absorption s aufgetragen sind. Für das oben genannte Beispiel eines Gasgemischs mit einem 7-prozentigen H₂-Anteil und einem 30-prozentigen CO₂-Anteil erhält man das dasselbe Referenzergebnis:
s (1000 mbar) = 0,2794 und s (1500 mbar) = 0,4055.

Man erkennt in Kalibrierfeld 21 zum einen die Änderung der Steigung der CO₂-Kennlinie mit steigendem Druck p sowie in dem unten gezeigten Zoom zum einen die größtenteils druckunabhängige Modifizierung des Signalbetrags durch die H₂-Konzentration. Dies ermöglicht es, die CO₂-Konzentration über den druckabhängigen Anstieg der gemessenen Absorption s und die H₂-Konzentration über den Betrag der gemessenen Absorption s zu ermitteln.

## Patentansprüche

1. Verfahren zur Bestimmung der Konzentration eines Messgases in einem Gasgemisch (1) durch Messung der Absorption von Licht (4) über den spektralen Bereich einer Absorptionslinie des Messgases, wobei das Gasgemisch (1) ein Quergas mit mindestens einer weiteren Absorptionslinie enthält, die die Absorptionslinie des Messgases überlappt und ein im Vergleich zu dieser unterschiedliches Druckverbreiterungsverhalten aufweist, wobei
- der Druck des Gasgemischs (1) variiert und die Messung der Absorption in Form von mindestens zwei Einzelmessungen bei unterschiedlichen Drücken des Gasgemischs (1) durchgeführt wird, **dadurch gekennzeichnet, dass**
- die Ergebnisse der Einzelmessungen mit von einem Kalibrierfeld (21) bereitgestellten Referenzergebnissen (22) verglichen werden, die zuvor durch Messung und/oder Simulation für unterschiedliche bekannte oder vorgegebene Drücke und Konzentrationen des Messgases und des Quergases ermittelt worden sind, und
- anhand des Vergleichs der mindestens zwei Einzelmessungen mit den Referenzergebnissen (22) die Konzentration des Messgases in dem Gasgemisch (1) bestimmt wird.

2. Verfahren nach Anspruch 1, **dadurch gekennzeichnet, dass** die von dem Kalibrierfeld (21) bereitgestellten Referenzergebnisse (22) Stützstellen bilden, zwischen denen für den Vergleich mit den mindestens zwei Einzelmessungen interpoliert wird.

3. Verfahren nach Anspruch 1 oder 2, **dadurch gekennzeichnet, dass** die Referenzergebnisse (22) durch Addition von ersten und zweiten Referenz-Zwischenergebnissen (21) berechnet werden, die von dem Kalibrierfeld (21) Sub-Kalibrierfeldern bereitgestellt werden, wobei
- die ersten Referenz-Zwischenergebnisse für unterschiedliche Drücke und unterschiedliche Konzentrationen des Messgases ohne Gegenwart des Quergases ermittelt worden sind und
- die zweiten Referenz-Zwischenergebnisse für unterschiedliche Drücke und unterschiedliche Konzentrationen des Quergases ohne Gegenwart des Messgases ermittelt worden sind.

4. Verfahren nach einem der vorangehenden Ansprüche, **dadurch gekennzeichnet, dass** die jeweils für dieselben Konzentrationen aber unterschiedliche Drücke ermittelten Referenzergebnisse (22) oder Referenz-Zwischenergebnisse in Form der Absorptionen bei den unterschiedlichen Drücken oder in Form der Absorption bei einem Druck und der Änderung der Absorption bei Änderung des Druckes bereitgestellt werden.

5. Verfahren nach einem der vorangehenden Ansprüche basierend auf der direkten Absorptionsspektroskopie.

6. Verfahren nach einem der Ansprüche 1 bis 4 basierend auf der Wellenlängenmodulationsspektroskopie.

7. Gasanalysator zur Bestimmung der Konzentration eines Messgases in einem Gasgemisch (1) durch Messung der Absorption von Licht (4) über den spektralen Bereich einer Absorptionslinie des Messgases, wobei das Gasgemisch (1) ein Quergas mit mindestens einer weiteren Absorptionslinie enthält, die die Absorptionslinie des Messgases überlappt und ein im Vergleich zu dieser unterschiedliches Druckverbreiterungsverhalten aufweist,
- mit einer wellenlängenabstimmbaren Lichtquelle (3), deren Licht (4) durch eine das Gasgemisch (1) aufnehmende Messküvette (2) auf einen Messdetektor (5) mit nachgeschalteter Auswerteeinrichtung (13) fällt, und
- mit einer Modulationseinrichtung (6), die zur wellenlängenabhängigen Abtastung der ersten Absorptionslinie die Wellenlänge des Lichts (4) der Lichtquelle (3) innerhalb periodisch aufeinanderfolgender Abtastintervalle variiert, wobei
- eine Druckeinstellanordnung (15) vorhanden ist, die dazu ausgebildet ist, mindestens zwei unterschiedliche Drücke des Gasgemischs (1) in der Messküvette (2) einzustellen,
- die Auswerteeinrichtung (13) ein Kalibrationsfeld (21) mit durch Messung und/oder Simulation für unterschiedliche bekannte oder vorgegebene Drücke und Konzentrationen des Messgases und des Quergases ermittelten Referenzergebnissen (22) für die Absorption enthält, und
- die Auswerteeinrichtung (13) dazu ausgebildet ist, durch Vergleich der Ergebnisse von bei mindestens zwei unterschiedlichen Drücken des Gasgemischs (1) durchgeführten Einzelmessungen der Absorption mit den von dem Kalibrierfeld (21) bereitgestellten Referenzergebnissen (22) die Konzentration des Messgases in dem Gasgemisch (1) zu bestimmen .

## Claims

1. Method for determining the concentration of a measurement gas in a gas mixture (1) by measuring the absorption of light (4) over the spectral range of an absorption line of the measurement gas, wherein the gas mixture (1) contains a cross gas with at least one further absorption line, which overlaps the absorption line of the measurement gas and has a pressure enlarging behaviour which differs by comparison herewith, wherein
- the pressure of the gas mixture (1) varies, and the measurement of the absorption is carried out in the form of at least two individual measurements with different pressures of the gas mixture (1), **characterised in that**
- the results of the individual measurements are compared with reference results (22) provided by a calibration field (21), said reference results having been ascertained in advance by measurement and/or simulation for different known or predetermined pressures and concentrations of the measurement gas and the cross gas, and
- the concentration of the measurement gas in the gas mixture (1) is determined on the basis of the comparison of the at least two individual measurements with the reference results (22) .

2. Method according to claim 1, **characterised in that** the reference results (22) provided by the calibration field (21) form sampling points, between which interpolation is carried out for the comparison with the at least two individual measurements.

3. Method according to claim 1 or 2, **characterised in that** the reference results (22) are calculated by adding first and second reference intermediate results (21) which are provided by the calibration field (21) to sub calibration fields, wherein
- the first reference intermediate results have been ascertained for different pressures and different concentrations of the measurement gas without the presence of the cross gas and
- the second reference intermediate results have been ascertained for different pressures and different concentrations of the cross gas without the presence of the measurement gas.

4. Method according to one of the preceding claims, **characterised in that** the reference results (22) or reference intermediate results ascertained for the same concentrations but different pressures in each case are provided in the form of the absorptions with the different pressures or in the form of the absorption with a pressure and the change in the absorption with the change in the pressure.

5. Method according to one of the preceding claims based on direct absorption spectroscopy.

6. Method according to one of claims 1 to 4 based on wavelength modulation spectroscopy.

7. Gas analyser for determining the concentration of a measurement gas in a gas mixture (1) by measuring the absorption of light (4) over the spectral range of an absorption line of the measurement gas, wherein the gas mixture (1) contains a cross gas with at least one further absorption line which overlaps the absorption line of the measurement gas and has a pressure enlarging behaviour which differs by comparison herewith,
- with a wavelength-tuneable light source (3), the light (4) of which strikes a measurement detector (5) with a downstream evaluation facility (13) through a measurement vessel (2) receiving the gas mixture (1), and
- with a modulation facility (6), which varies the wavelength of the light (4) of the light source (3) within periodically consecutive sampling intervals in order to sample the first absorption line in a wavelength dependent manner,
wherein
- a pressure setting arrangement (15) exists, which is embodied to set at least two different pressures of the gas mixture (1) in the measuring vessel (2),
- the evaluation facility (13) contains a calibration field (21) with reference results (22) for the absorption which are ascertained by measurement and/or simulation for different known or predetermined pressures and concentrations of the measurement gas and the cross gas, and
- the evaluation facility (13) is embodied to determine the concentration of the measurement gas in the gas mixture (1) by comparing the results of individual measurements of the absorption carried out with at least two different pressures of the gas mixture (1) with the reference results (22) provided by the calibration field (21).

## Revendications

1. Procédé de détermination de la concentration en un gaz à mesurer dans un mélange (1) gazeux par mesure de l'absorption de la lumière (4) sur un domaine spectral d'une raie d'absorption du gaz à mesurer, dans lequel le mélange (1) gazeux contient un gaz transversal ayant au moins une autre raie d'absorption, qui chevauche la raie d'absorption du gaz à mesurer et qui a, par rapport à celle-ci, un comportement différent d'élargissement sous l'effet de la pression, dans lequel
- la pression du mélange (1) gazeux varie et on effectue la mesure de l'absorption sous la forme d'au moins deux mesures individuelles à des pressions différentes du mélange (1) gazeux, **caractérisé en ce que**
- on compare les résultats des mesures individuelles à des résultats (22) de référence, qui sont mis à disposition par un champ (21) d'étalonnage et qui ont été déterminées auparavant par une mesure et/ou par une simulation pour des pressions et concentrations du gaz de mesure et du gaz transversal différentes ou connues à l'avance, et
- on détermine la concentration en le gaz à mesurer du mélange (1) gazeux, à l'aide de la comparaison des au moins deux mesures individuelles aux résultats (22) de référence.

2. Procédé suivant la revendication 1, **caractérisé en ce que** les points d'appui entre lesquels on interpole, pour la comparaison avec les au moins deux mesures individuelles, forment les résultats (22) de référence mis à disposition par le champ (21) d'étalonnage.

3. Procédé suivant la revendication 1 ou 2, **caractérisé en ce que** l'on calcule les résultats (22) de référence par addition de premiers et de deuxièmes résultats (21) intermédiaires de référence, qui sont mis à disposition par le champ (21) d'étalonnage en des sous-champs d'étalonnage, dans lequel
- on a déterminé les premiers résultats intermédiaires de référence pour des pressions différentes et des concentrations différentes du gaz à mesurer sans présence du gaz transversal, et
- on a déterminé les deuxièmes résultats intermédiaires de référence pour des pressions différentes et des concentrations différentes du gaz transversal sans présence du gaz à mesurer.

4. Procédé suivant l'une des revendications précédentes, **caractérisé en ce que** l'on met à disposition les résultats (22) de référence ou les résultats intermédiaires de référence déterminés respectivement pour les mêmes concentrations mais des pressions différentes sous la forme des absorptions aux pressions différentes ou sous la forme de l'absorption à une pression et de la variation de l'absorption lorsque la pression varie.

5. Procédé suivant l'une des revendications précédentes, reposant sur la spectroscopie d'absorption directe.

6. Procédé suivant l'une des revendications 1 à 4, reposant sur la spectroscopie de modulation en longueurs d'onde.

7. Analyseur de gaz pour la détermination de la concentration en un gaz à mesure d'un mélange (1) gazeux, par mesure de l'absorption de la lumière (4) sur le domaine spectral d'une raie d'absorption du gaz à mesurer, dans lequel le mélange (1) gazeux contient un gaz transversal ayant au moins une autre raie d'absorption, qui chevauche la raie d'absorption du gaz à mesurer et qui a, par rapport à celle-ci, un comportement différent d'élargissement sous l'effet de la pression,
- comprenant une source (3) lumineuse accordable en longueur d'onde, dont la lumière (4) tombe, en passant dans une cuvette (2) de mesure recevant le mélange (1) gazeux, sur un détecteur (5) de mesure ayant un dispositif (13) d'analyse en aval, et
- comprenant un dispositif (6) de modulation qui, pour l'échantillonnage, en fonction de la longueur d'onde, de la première raie d'absorption, fait varier la longueur d'onde de la lumière (4) de la source (3) lumineuse dans des intervalles d'échantillonnage se succédant périodiquement, dans lequel
- il y a un dispositif (15) de réglage de la pression, qui est constitué pour établir deux pressions différentes du mélange (1) gazeux dans la cuvette (2) de mesure,
- le dispositif (13) d'analyse comporte un champ (21) d'étalonnage ayant des résultats (22) de référence de l'absorption déterminés par une mesure et/ou une simulation pour des pressions et des concentrations en le gaz de mesure et en le gaz transversal différentes connues ou données à l'avance, et
- le dispositif (13) d'analyse est constitué pour déterminer la concentration en le gaz à mesurer du mélange (1) gazeux, par comparaison des résultats de mesures individuelles de l'absorption effectuées à au moins deux pressions différentes du mélange (1) gazeux à des résultats (22) de référence mis à disposition par le champ (21) d'étalonnage.
